# EUROPÄISCHE PATENTANMELDUNG

(11) **EP 0 866 061 A1**
(43) Veröffentlichungstag der Anmeldung: **23.09.1998**
(21) Anmeldenummer: 98102786.5
(22) Anmeldetag: 18.02.1998
(51) Int. Cl.: C07D 239/06

(54) **Verfahren zur Abtrennung von Pyrimidinderivaten aus wässrigen Lösungen**

(30) Priorität: 18.03.1997 DE 19711082
(71) Anmelder: Degussa Aktiengesellschaft, 60311 Frankfurt (DE)
(72) Erfinder: Stockhammer, Stefan, 97662 Brusno (SK); Treffenfeldt, Wiltrud, Professor, 63179 Obertshausen (DE); Preuss, Andrea, Dr., 63456 Hanau (DE)

(57) **Zusammenfassung**

Die Erfindung betrifft ein Verfahren zur Abtrennung und Aufreinigung von Pyrimidinderivaten, besonders Tetrahydropyrimidinen, insbesondere 2-Methyl-1,4,5,6-Tetrahydropyrimidin-4-Carbonsäuren (Ectoine)aus wäßrigen Lösungen durch Adsorption an anorganischen Feststoffen und eine damit kombinierte Crossflow-Filtration.

## Beschreibung

Die Erfindung betrifft ein Verfahren zur Abtrennung und Aufreinigung von Pyrimidinderivaten, besonders von Tetrahydropyrimidinen, insbesondere 2-Methyl-1,4,5,6-tetrahydropyrimidin-4-Carbonsäuren (Ectoine)aus wäßrigen Lösungen durch Adsorption an Zeolithen und eine damit kombinierte Cross-Filtration.

Mit Tetrahydropyrimidinderivaten sind insbesondere die aus der EP-A1-0553884 bekannten Verbindungen gemeint.

Strukturell sind Ectoine cyclische Aminosäurederivate, die zur Klasse der sogenannten 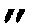 kompatiblen Solute" gehören. Sie sind auch in hoher Konzentration mit dem Cytoplasma kompatibel und stabilisieren die Zellkomponenten in einem Milieu geringer Wasseraktivität. Diese Wirkung weist auf ein breites Anwendungsfeld in medizinischen und kosmetischen Bereichen hin.

Durch neue biotechnische Verfahren ist es gelungen, halophile Eubakterien z. B. der Gattung Halomonas zu züchten und diese Organismen zum Ausscheiden der Ectoine in das sie umgebende Medium zu veranlassen (T. Sauer et al. GIT Fachz. Lab. 10/95).

In dem sich anschließenden Aufarbeitungsverfahren wird das Ectoin dann über organische Kationenaustauscher und Kristallisation gereinigt.

Es hat sich jedoch herausgestellt, daß es sich dabei um ein vielstufiges und sehr zeitaufwendiges Verfahren handelt, wenn man zu dem gängigen Verkaufsprodukt mit einer Reinheit von ca. 90% gelangen will.

Aufgabe der Erfindung ist es, ein alternatives Verfahren zur Verfügung zu stellen, das eine effektive Abtrennung der Tetrahydropyrimidinderivate gemäß EP-A1 - 0553 884 und insbesondere Ectoine aus einer gegebenenfalls weitere organische Verbindungen enthaltenden wäßrigen Lösung, insbesondere Fermentationslösungen, und die Aufreinigung dieser Pyrimidinderivate beinhaltet, soweit sie an Zeolithen adsorbierbar sind.

Gegenstand der Erfindung ist insbesondere ein Verfahren zur Abtrennung und Aufreinigung von Pyrimidinderivaten, besonders von Tetrahydropyrimidinen, insbesondere von 2-Methyl-1,4,5,6-Tetrahydropyrimidin-4-Carbonsäuren (Ectoine) aus wäßrigen Lösungen, dadurch gekennzeichnet, daß man die wäßrigen Lösungen bei einem pH-Wert von 1,5 bis 7,0 mit einem bevorzugt sauren Zeolith in Kontakt bringt, der einen Modul von 15 bis 1000 besitzt, nach erfolgter Adsorption den belegten Zeolith abtrennt, und die adsorbierten Derivate mit einer wäßrigen, auf einen pH-Wert >8,0 eingestellten Lösung, insbesondere Ammoniumhydroxid-haltig, desorbiert.
Man kann die Lösung auch durch Zusatz einer basischen organischen Komponente, insbesondere Lysin, auf diesen pH-Wert einstellen.
(Unter Modul versteht man das molare Verhältnis von SiO₂ zu Al₂O₃).

In Abhängigkeit von den an die Reinheit der zu isolierenden Derivate gestellten Ansprüchen ist dieser Verfahrensschritt gegebenenfalls mehrfach durchzuführen.

Das bedeutet, daß man die durch Desorption nach dem ersten Desorptionsschritt gewonnene Lösung zusätzlich ein- oder mehrfach in dem für eine effektive Adsorption der Derivate geeigneten pH-Wert-Bereich mit den geeigneten Zeolithtypen in Kontakt bringt und, wenn es sich um eine mehrfach ablaufende Reinigungssequenz handelt, entsprechend häufig die adsorbierten Derivate desorbieren läßt.

Unter den erfindungsgemäß abzutrennenden und aufzureinigenden Tetrahydropyrimidinderivaten versteht man insbesondere Verbindungen der Formel 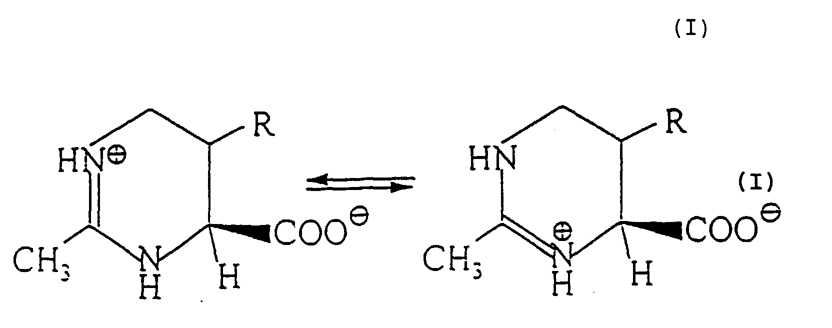 mit der Bedeutung für R: H, OH, insbesondere H und die aus der EP-A1-0533 884 bekannten adsorbierbaren Tetrahydropyrimidinderivate.

Die Lösungen, aus denen man diese und die Ectoine gemäß Formel (I) abzutrennen hat, sind im allgemeinen wäßriger Natur.

Bevorzugt fallen die aufzureinigenden Lösungen nach der Fermentation mit geeigneten Bakterien an. Der pH-Wert ist dann den Adsorptionsbedingungen entsprechend anzupassen.

In einer bevorzugten Ausführungsform werden die Mikroorganismen in diesem Fall zumindest teilweise zuvor aus der Fermentationsbrühe entfernt, insbesondere durch Abzentrifugieren, möglich ist jedoch auch eine direkte Aufreinigung der Ectoine aus der Biomasse-haltigen Lösung.

Durch geeignete Aufschlußmethoden (hypoosmotischer Schock, mechanischer oder chemischer Zellaufschluß) erfolgt eine Freisetzung des Ectoins aus den Zellen.

Vorteilhaft fällt man die die Abtrennung der Ectoine durch Adsorption gegebenfalls störenden Proteine durch eine geeignete Einstellung des pH-Werts aus und trennt sie dann ab.

Für die Adsorption der erfindungsgemäß abzutrennenden Verbindungen geeignete Zeolithe sind solche der Typen, Y, DAY, Mordenit, dealuminierter Mordenit, ZSM-5, dealuminierter ZSM-5, β oder VPI5 und einem Modul von 10 bis 1000, insbesondere von 15 bis 200, bevorzugt von 15 bis 45. Bevorzugt setzt man den ZSM-5-Typ der H-, Ammonium- oder Na-Form ein.

Das Verfahren läuft in einem Temperaturbereich zwischen 15 und 80°C ab.

Im allgemeinen bewegt sich die Konzentration der Ectoine bis zur jeweiligen Löslichkeitsgrenze im wäßrigen Bereich, insbesondere von 0,1 bis 4,0 g/l. Je nach Bereich werden die Adsorbentien pulverförmig oder als Formkörper eingesetzt.

In einer vorteilhaften Ausführungsform kombiniert man die Adsorption mit einer Crossflow-Filtration, wobei man zuvor die Tetrahydropyrimidinderivate, insbesondere die Ectoine enthaltenden Lösungen mit Suspensionen eines sauren Zeoliths in Kontakt bringt, und nach der Adsorption dieser Derivate (A)
a) den so beladenen Zeolith an einer porösen Fläche/Membran vorbeiströmen läßt, wobei man
b) eine Druckdifferenz zwischen der Überström- und der gegenüberliegenden Seite der Fläche/Membran einstellt, so daß
c) ein Teil der die Fläche/Membran überströmenden von den Derivaten ganz oder teilweise befreiten Lösung die Fläche/Membran quer zur Fließrichtung durchströmt (Filtratfluß),
d) in einem Waschschritt die von den Verbindungen (A) befreite Lösung, die gegebenenfalls organische Verunreinigungen enthält, abtennt, und
e) anschließend die adsorbierten Verbindungen desorbiert.

Letzteres geschieht bei einem geeigneten pH-Wert, insbesondere bei einem pH-Wert > 8,0, oder mit einem geeigneten Lösungsmittel, z. B. Methanol.

Als Adsorbentien besonders geeignet sind feinteilige Zeolithpulver der oben genannten Typen mit einem Partikeldurchmesser von 1 bis 100 µm, insbesondere 2 bis 20 µm.

Diese werden im allgemeinen in Form von wäßrigen Suspensionen mit einem Feststoffgehalt von 5 bis 60 Gew.%, bevorzugt 20 bis 50 Gew.% eingesetzt.

Als Filterflächen werden organische oder keramische Membranen mit Ultra-, Nano- oder Mikrofiltrationseigenschaften verwendet.

Führt man das Verfahren erfindungsgemäß durch, bilden sich auf dem Filtermedium keine Deckschichten, die zu hohen Filtrationswiderständen führen. Damit vermeidet man ein Problem, das bei der Anwendung dieses Verfahrens in anderen Bereichen über zusätzliche Reinigungszyklen für die verwendete Membran gelöst werden muß (DE-PS 43 32 175).

Man erhält erfindungsgemäß erstaunlicherweise bereits in der ersten Reinigungsstufe ein Produkt mit einer Reinheit von >70 %, die sich in einem zweiten analog verlaufenden Reinigungsschritt auf über 90 % steigern läßt.

Zur Erhöhung der Reinheit kann man die gemäß Anspruch 10 (Schritt e) erhaltenen Lösungen ein- oder mehrfach in das erfindungsgemäße Reinigungsverfahren einschleusen. Eine Variante des erfindungsgemäßen Verfahrens besteht darin, dieses mit der aus dem Stand der Technik bekannten Reinigung z. B. von Ectoinlösungen durch Verwendung von organischen Kationenaustauschern und anderen Reinigungsschritten zu kombinieren.

Ein entsprechender Adsorptions- und Desorptionsschritt erfolgt vor und/oder nach einer Reinigungsstufe durch Adsorption an einem der erfindungsgemäß verwendeten Zeolithe, der sich eine Desorption angeschlossen hat.

### Beispiel 1

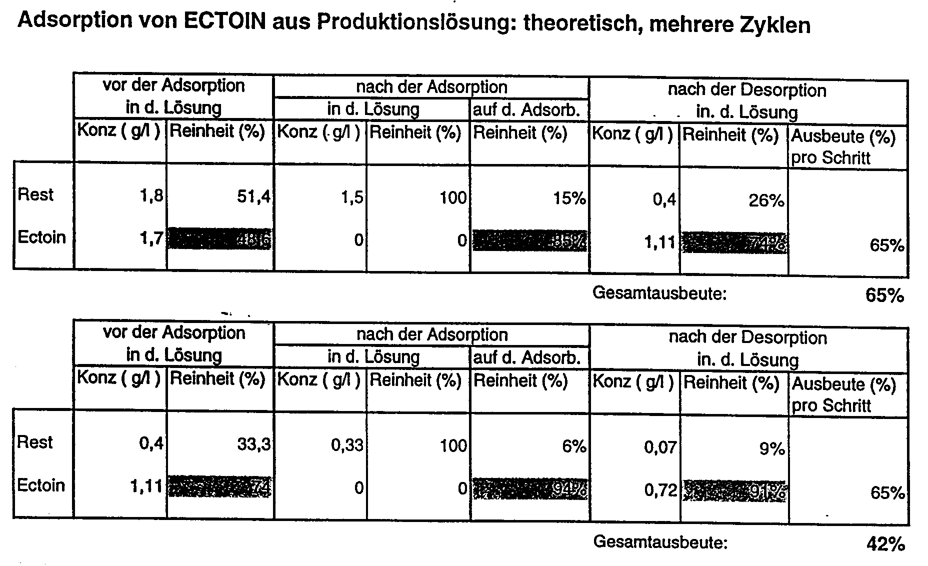

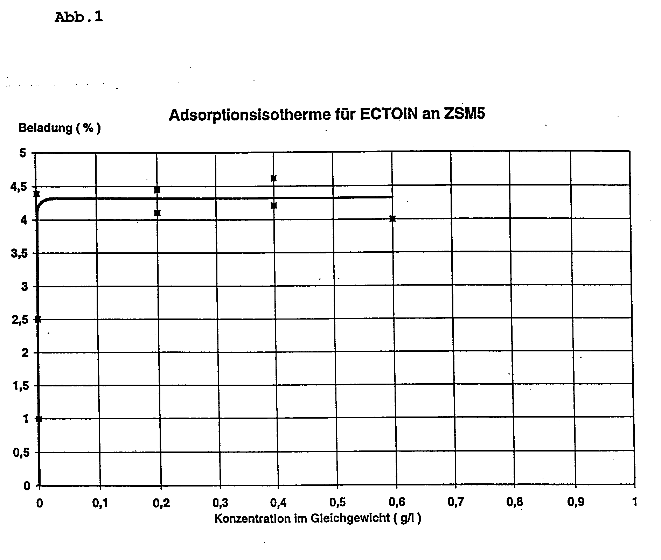

## Patentansprüche

1. Verfahren zur Abtrennung und Reinigung von Pyrimidinderivaten, insbesondere Tetrahydropyrimidinen, aus wäßrigen Lösungen,
**dadurch gekennzeichnet,**
daß man die Lösungen mit einem sauren Zeolith in Kontakt bringt und nach erfolgter Adsorption der Derivate bei einem geeigneten pH-Wert diese durch Desorption bei einen anderen pH Wert zurückgewinnt.

2. Verfahren gemäß Anspruch 1,
**dadurch gekennzeichnet,**
daß man die wäßrigen Lösungen bei einem pH-Wert von 1,0 bis 7,0 mit einem sauren Zeolith in Kontakt bringt, der einen Modul von 15 bis 1000 besitzt und nach erfolgter Adsorption die adsorbierten Derivate mit einer wäßrigen, auf einen pH Wert >8,0 gegebenenfalls durch Zusatz einer basischen organischen Komponente eingestellten Lösung desorbiert.

3. Verfahren gemäß den Ansprüchen 1 und 2
**dadurch gekennzeichnet,**
daß man 2-Methyl-1,4,5,6- Tetrahydropyrimidin-4-Carbonsäuren (Ectoine) der allgemeinen Formel 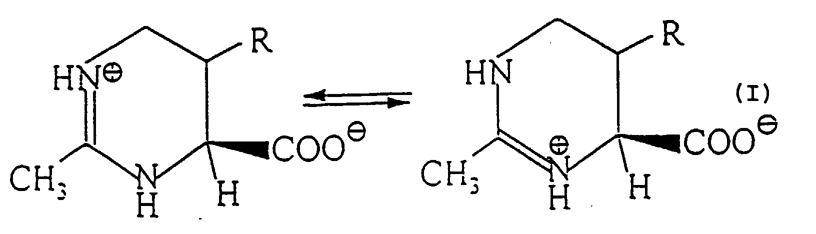 mit der Bedeutung für R: H, OH, aus wäßrigen Lösungen abtrennt

4. Verfahren gemäß den Ansprüchen 1 bis 3,
**dadurch gekennzeichnet,**
daß man die durch Desorption erhaltenen Lösung erneut mit einem der oben genannten Zeolithtypen in Kontakt bringt, die adsorbierten Derivate desorbiert und diese Schritte gegebenenfalls ein- oder mehrfach wiederholt.

5. Verfahren gemäß den Ansprüchen 1 bis 4,
**dadurch gekennzeichnet,**
daß man als wäßrige Lösung eine Fermentationslösungen einsetzt.

6. Verfahren gemäß Anspruch 5,
**dadurch gekennzeichnet,**
daß man vor der Adsorption die Mikroorganismen. zumindest teilweise aus der Fermentationslösung abtrennt.

7. Verfahren gemäß den Ansprüchen 5 und 6,
**dadurch gekennzeichnet,**
daß man vor der Adsorption die löslichen Proteine zumindest teilweise aus der Fermentationslösung abtrennt.

8. Verfahren gemäß einem oder mehreren der Ansprüche 1 bis 7,
**dadurch gekennzeichnet,**
daß man als Adsorbentien saure Zeolithe der Typen Y, DAY, Mordenit, dealuminierter Mordenit, ZSM-5, dealuminierter ZSM-5, β oder VPI-5 mit dem Modul von 15 bis 1000 einsetzt.

9. Verfahren gemäß Anspruch 7,
**dadurch gekennzeichnet,**
daß man Zeolithe vom Typ ZSM5 oder Mordenit in der H-, Ammonium oder Na-Form einsetzt.

10. Verfahren gemäß einem oder mehreren der Ansprüche 1 bis 9 zur Abtrennung von an feinteiligen Zeolithen adsorbierten Pyrimidinderivaten, insbesondere gemäß Formel (I), (A),
**dadurch gekennzeichnet,**
daß man es mit einer Crossflow-Filtration kombiniert, wobei man
a) die mit (A) beladenen Zeolithe in Form von Susppensionen an einer porösen Fläche/Membran vorbeiströmen läßt, wobei man
b) eine Druckdifferenz zwischen der Überström- und der gegenüberliegenden Seite der Fläche/Membran einstellt, so daß
c) ein Teil der die Fläche/Membran überströmenden von den adsorbierten Verbindungen (A) ganz oder teilweise befreiten Lösung die Fläche/Membran quer zur Fließrichtung durchströmt (Filtratfluß)
d) in einem Waschschritt die von den Verbindungen (A) befreite Lösung, die gegebenenfalls organische Verunreinigungen enthält, abtrennt, und
e) anschließend die adsorbierten Verbindungen (A) desorbiert.

11. Verfahren gemäß Anspruch 10,
**dadurch gekennzeichnet,**
daß man Zeolithe mit einem mittleren Partikeldurchmesser von 1 bis 100 µm einsetzt.

12. Verfahren gemäß den Ansprüchen 10 und 11,
**dadurch gekennzeichnet,**
daß man einen Transmembrandruck von 0,2 bis 3 bar einstellt.

13. Verfahren gemäß den Ansprüchen 10 bis 12,
**dadurch gekennzeichnet,**
daß man keramische oder organische Membranen/poröse Flächen mit Ultrafiltrations- oder Mikro- oder Nanofiltrationseigenschaften verwendet.

14. Verfahren gemäß einem oder mehreren der vorhergehenden Ansprüche,
**dadurch gekennzeichnet,**
daß man die desorbierte Lösung ein- oder mehrfach der Adsorption und Desorption unterwirft.

15. Verfahren gemäß einem oder mehreren der vorhergehenden Ansprüche,
**dadurch gekennzeichnet,**
daß man das Verfahren zur Abtrennung von Verbindungen (A) mit der Adsorption an Kationenaustauschern oder anderen Reinigungsschritten und den daraus durch Desorption gegebenfalls wiederholt gewonnenen Lösungen kombiniert.
